# EUROPEAN PATENT APPLICATION

(11) **EP 4 458 305 A1**
(43) Date of publication of application: **06.11.2024**
(21) Application number: 24173117.3
(22) Date of filing: 29.04.2024
(51) Int. Cl.: A61B 34/20, A61B 34/32

(54) **VIEW SHUTTLING IN ROBOTIC STEERING OF A CATHETER**

(30) Priority: 01.05.2023 US 202318310004
(71) Applicant: Siemens Medical Solutions USA, Inc., Malvern, PA 19355 (US)
(72) Inventor: COLLINS, Jarrod, Wilsonville, OR, 97070 (US); KIM, Young-Ho, West Windsor, NJ, 08550 (US); MANSI, Tommaso, Plainsboro, NJ, 08536 (US)
(74) Representative: Horn Kleimann Waitzhofer Schmid-Dreyer Patent- und Rechtsanwälte PartG mbB

(57) **Abstract**

For robotically operating a catheter (450), a robotic catheter system (400) controls navigation along a trajectory (202, 204, 206). The trajectory (202, 204, 206) defines the movement (120), allowing the robotic catheter system (400) to navigate the catheter (450) with less or no user intervention. The catheter (450) is controlled in a way allowing the operator focus less on the catheter (450), avoiding or limiting parking or retracting.

## Description

### BACKGROUND

The present embodiments relate to robotic control of a medical catheter. One example medical catheter is an intracardiac echocardiography (ICE) catheter, which is used for imaging in cardiac interventional and diagnostic procedures. ICE can provide close feedback of anatomical structures and tools during a surgical procedure. Other catheters include ablation, optical imaging, guidance, stent placement, needle, or therapeutic.

ICE catheters require significant training to learn to steer and manipulate for desired cardiac anatomical views. Similarly, the interventionalist is commonly manipulating multiple catheters (e.g., therapeutic devices, sensors, and the ICE imaging) throughout the procedure. Many interventionalists have adopted the habit of retracting or parking the ICE imaging catheter during therapeutic delivery. This underutilizes the potential of ICE. Early identification of life-threatening complications can be observed from standard views throughout the heart. However, by retracting or parking the ICE catheter, the ability of ICE for monitoring complications or other active processes is self-limited.

Robotics may assist the operator. Simple manual, but remote control, of a robotic catheter system may suffer from similar problems as non-robotic operation unless an additional person is used to control. Manual operation through robotics may be poorly reproducible for imaging. The robotics may rely on electromagnetic (EM) field sensing in the catheter for closed-loop control, aiding in navigation of the ICE catheter. The control still relies on the interventionalist for where and when to navigate. Many ICE catheters do not include the needed EM sensors.

### SUMMARY

By way of introduction, the preferred embodiments described below include methods, systems, and robots for robotically operating catheters. The robotic catheter system controls navigation along a trajectory. The trajectory defines the movement, allowing the robotic catheter system to navigate the catheter with less or no user intervention. The catheter is controlled in a way allowing the operator focus less on the catheter, avoiding or limiting parking or retracting.

In a first aspect, a method is provided for robotic control of an imaging catheter. A trajectory of catheter movement within a patient is acquired. A robotic catheter system moves the catheter along the trajectory. An image from the imaging catheter of the patient while the imaging catheter is on the trajectory is displayed.

According to one implementation, the trajectory is acquired by receiving an input of a start point and an input of an end point from an operator navigating the imaging catheter along the trajectory with the robotic catheter system. The navigation for moving the imaging catheter along the trajectory can then repeat between the start point and end point without the operator performing the navigation.

In another implementation, the trajectory is stored during manual control by an operator of the robotic catheter system. For example, the trajectory is stored as motor positions or imaging catheter positions during the manual control.

In yet another implementation, a user designation of the trajectory is received. For example, user input of way points within a boundary are received on a graphics user interface.

As another implementation, the imaging catheter is moved to waypoints on the trajectory in response to activation for each of the waypoints by the user. An interval of the waypoints input by an operator. In one further implementation, one or more waypoints are interpolated from others of the waypoints.

According to an implementation, the robotic catheter system moves the imaging catheter by over the trajectory multiple times. In other implementations, the imaging catheter movement includes reversing or progressing along the trajectory in response to operator input. For example, the movement reverses to a point along the trajectory having a view indicated by an operator.

In an implementation, the robotic catheter system moves the catheter in synchronization with heart motion.

As another implementation, a user selects the trajectory from a library of trajectories.

In a second aspect, a control system is provided for a steerable catheter. A robotic system is provided for the operation of a steerable catheter. A memory is configured to store a trajectory along which the robotic system navigates the steerable catheter. A processor is configured to cause the robotic system to navigate the steerable catheter in a rewind along the trajectory.

In an implementation, the processor is configured to cause the navigation along the trajectory in steps. A step size is configured by input from an operator and the navigation for each step is in response to user activation.

In another implementation, the steerable catheter is an imaging catheter where a sequence of images is generated as the robotic system navigates the imaging catheter along the trajectory. The processor is configured to cause the robotic system to navigate the imaging catheter to a point along the trajectory associated with one of the images in the sequence.

As one implementation, the processor is configured to store the trajectory in the memory as an operator controls the robotic system. As another implementation, the processor is configured to store the trajectory in the memory where the trajectory is created with a user interface receiving input of waypoints within a boundary.

In a third aspect, a catheter control system is provided. A memory is configured to store instructions. A processor is configured by the instructions to control a catheter robot to move an intracardiac echocardiography catheter along a previously established trajectory.

In one implementation, the processor is configured to control the catheter robot to move the intracardiac echocardiography catheter in reverse along the trajectory as a rewind to a previous view.

The present invention is defined by the following claims, and nothing in this section should be taken as a limitation on those claims. Features of one aspect or type of claim (e.g., method or system) may be used in other aspects or types of claims. Further aspects and advantages of the invention are discussed below in conjunction with the preferred embodiments and may be later claimed independently or in combination.

### BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.
Figure 1 is a flow chart diagram of one embodiment of a method for robotically operating a catheter with shuttling along a trajectory;
Figure 2 illustrates different example trajectories from manual operation of the robotic catheter system;
Figure 3 illustrates an example trajectory from user input of waypoints within a boundary; and
Figure 4 is a block diagram of one embodiment of a system for shuttle control of a robotic catheter system.

### DETAILED DESCRIPTION OF THE DRAWINGS AND PRESENTLY PREFERRED EMBODIMENTS

Local view shuttling is provided for robotic catheter operation, such as operation of an ICE or other imaging catheter. A trajectory of robotic control inputs that cause the imaging catheter to survey across a 3D image feature is created and may be iterated or used to navigate. An automated or semi-automatic steering protocol provides local view shuttling based on a priori motor configuration knowledge of the trajectory. Robotic control history is applied to provide repeatable motion along a pre-defined imaging trajectory for ICE clinical workflows. Path generation and/or motion-control automation are provided for surveying of anatomy.

Discrete commands may be given to (1) advance step-by-step forward or backward within that trajectory or (2) automatically cycle through the trajectory at a defined interval. The assistive robotic controller holds and/or actively manipulates the ICE catheter, either through manual input or semi-autonomy, easing the workload of the interventionalist during treatment and enabling the use of ICE for complex procedures.

The assistive ICE robotic controller provides automation or semi-automation for repetitive movement along a pre-defined imaging trajectory. For example, physicians typically survey anatomy with ICE to view how devices interact or have deployed over a region. This surveying may be performed multiple times with continuous back-and-forth between manipulating the ICE to survey and adjust a device based on ICE feedback. The motion of the initial survey is reproduced without full control by the operator, allowing for hands-free repeat image surveying of anatomy and device deployment. As another example, physicians survey the anatomy to determine their preferred view for parking before performing a therapeutic task. During this survey, the user may pass a desired view. It may be difficult to return to the desired view by hand or manual control. Rather than compromising for a less optimal view, the trajectory is used to rewind the survey to the preferred view. As yet another example, certain techniques (i.e., mapping) are used to generate a 3D map of the heart chambers for procedural guidance. These mapping techniques can have error depending on sampling rate compared to cardiac motion. The trajectory is used to more accurately step through the mapping process and better account for heart motion (e.g., moving and image gating in synchronization with the heart cycle and/or deformation correction based on the heart cycle).

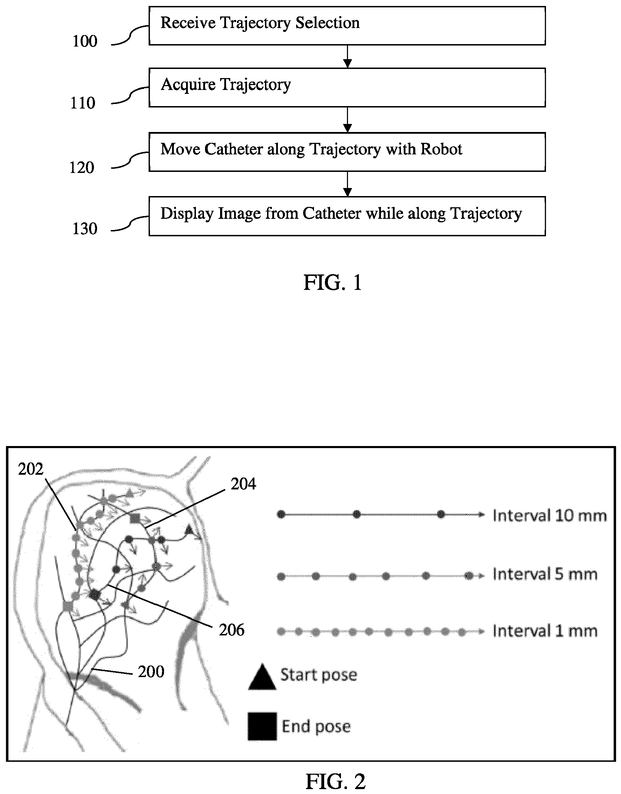

Figure 1 is a flow chart diagram of one embodiment of a method for robotic control of a catheter, such as an imaging catheter (e.g., ICE catheter). A trajectory is acquired and used for processor-based control of the robotic catheter system. The catheter is navigated along the trajectory, such as a loop survey or rewind. The catheter shuttles along the trajectory so that the operator is freed for other activities in the procedure.

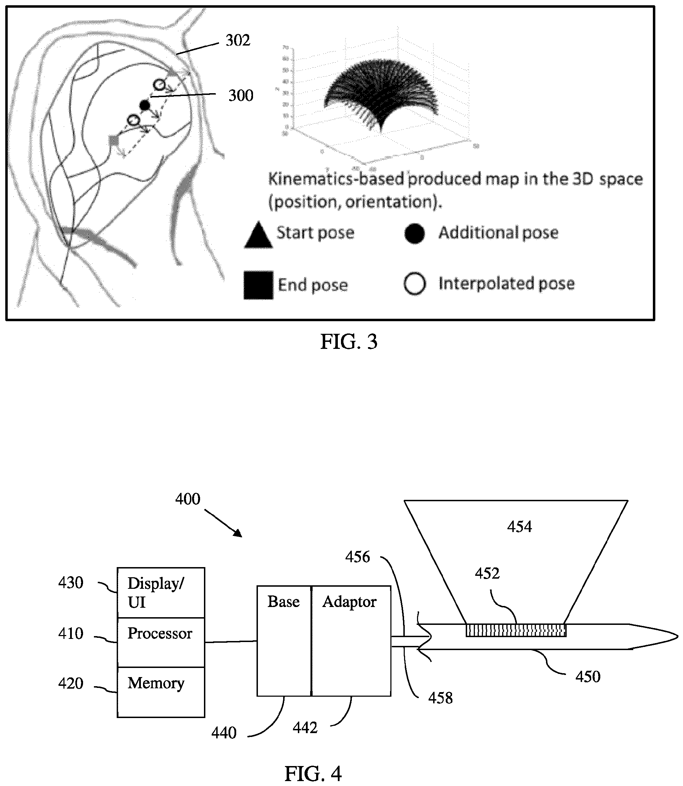

The method is implemented by the system and/or robotic system of Figure 4 or another system. A processor receives the selection, acquires the trajectory, and causes movement of the catheter using the trajectory. The robotic catheter system moves the catheter. An imaging system uses an array or other sensor on the catheter to generate and display an image of the patient.

Additional, different, or fewer acts may be provided. For example, act 100 and/or 130 are not provided. As another example, acts for the catheterization workflow are provided. In yet another example, acts for preoperative planning and/or pre, during, and/or post procedure imaging are provided. As another example, acts for ablation, stent placement, or another catheter function are provided.

The acts are performed in the order shown (top to bottom or numerical) or a different order. For example, act 130 occurs during act 120 (e.g., simultaneously or in real time).

In act 100, a user interface and/or processor receives user selection of a trajectory from a library of trajectories. The selection may be received from memory, such as where a default selection is made. Other sources and/or recipient devices may be provided.

The trajectory is a path of movement along the degrees of freedom of the catheter. For example, four degrees of freedom are provided: translation along a longitudinal axis of the catheter, rotation about the longitudinal axis, anterior-posterior bend, and left-right bend. In other examples, six degrees of freedom in a patient-centric space is provided. The trajectory path is continuous or a series of two or more samples or points. The trajectory has a start and end, with no, one, two, or more waypoints or samples in between. The trajectory may be defined with respect to any movement within the degrees of freedom, such as rotation, translation, bend in one plane, bend in another plane, or combinations thereof. For example, the trajectory has translation along a curve in three dimensions with rotation and/or bending being different or the same at different points or samples along the path or curve. A straight trajectory or a trajectory with no translation (e.g., rotation and/or bending only) may be provided.

A library of trajectories may be provided. The library is a set of one or more trajectories. Trajectories for different purposes (e.g., ablation monitoring, stent placement monitoring, other treatment monitoring, mapping, or surveying), patient characteristics (e.g., size or shape of the heart chamber), and/or catheter characteristics (e.g., purpose of the catheter, flexibility, degrees of freedom, or length) are provided. Different trajectories may be provided for different operators (interventionists), such as to reflect user preferences. The user may select the appropriate trajectory from the library. The user interface provides a list for selection. Alternatively, a branching menu is provided (e.g., separate selections of user, purpose, patient, and catheter in any order). Other menus for selection of trajectories may be provided. Alternatively, a default trajectory is used. A given trajectory may have different settings to account for patient, user, and/or catheter use rather than having different trajectories.

In another approach, no selection is provided. Instead, the use of the robotic catheter system for a patient is monitored or recorded and defines a trajectory. Alternatively, a single trajectory is used.

In one implementation, after a library, S, of trajectories, t, has been constructed or accessed in an exploration stage, the user initiates a shuttling stage. The user identifies a specific trajectory 't' from the library `S.' The user may identify one or more characteristics for the selected trajectory, t, such as indicating a step size (e.g., a size of interval `I' (i.e., the scale of single step size motions, up to sub-millimeter)). In other implementations, the trajectory is continuous without steps or intervals. Other characteristics of the trajectory may be selected or set.

In act 110, the processor (e.g., robotic catheter system controller) acquires a trajectory of catheter movement within a patient. The selected trajectory is loaded from memory, received at a user interface, and/or acquired by monitoring. In general, the trajectory is acquired based on operation of the robotic catheter system during use in the patient and/or loading of a generic trajectory, user created trajectory, and/or previously used trajectory.

For acquisition from operation for the current patient, the trajectory is acquired by storage of the trajectory during manual control by an operator of the robotic catheter system. For example, the operator surveys anatomy and/or other devices in the patient by controlling the robotic catheter system to move the catheter over a path. This path is recorded as the trajectory.

In one implementation, the user indicates the trajectory more specifically. The user may clip or designate the trajectory from the previous path. Alternatively, the user designates the trajectory while operating the robotic catheter system. For example, the processor receives an input designating a start point (e.g., activate a start key with the catheter at the start of the trajectory) and an input of an end point (e.g., activate an end key with the catheter at the end of the trajectory). As the operator navigates the catheter (e.g., imaging catheter) from the indicated start point to the to-be indicated end point, the movement and/or position of the catheter along the path is monitored and recorded as the trajectory. The trajectory is formed from an operator navigating the imaging catheter along the trajectory with the robotic catheter system. The user may indicate the interval for recording, such as forming the trajectory as sample points along the path at a user defined interval in time and/or position. Any user input to designate the start, end, and/or interval may be used, such as a button, graphic user input, and/or record function.

As an example, the user would like to survey an area of anatomy with an ICE catheter and at a later timepoint reproduce this survey. The ICE catheter begins at some location within the heart anatomy, A. The user then calls upon a software application to begin recording their motions (e.g., the motions of the catheter and/or robotic catheter system). The application labels the robotic motor configuration associated with the current location, A, as the starting point of the trajectory. The user then provides manual input (e.g., via joystick) to the robotic controller, moving the ICE catheter and surveying across the anatomical region of interest. Each input provided by the user to the robotic controller is recorded. Upon completion of the survey, the ICE catheter has now ended at some new point, B. The user provides input to the application that the survey has been completed (e.g., by joystick, voice, button, etc.).

This survey or initial use of the catheter along a trajectory is an exploration stage. The user controls the robot using the user interface (e.g., joystick, keyboards, or voice). The robotic catheter system continuously or periodically constructs a topological graph including the robot configuration states, 3-DOF position, and 3-DOF orientation based on kinematics. Within this mapping, the robotic catheter system indexes the trajectory. During the exploration stage, the user gives two inputs to save a trajectory. The trajectory is a sequential graph without additional branches. Thus, the user gives 'start' and 'end' inputs to save a single trajectory. A branching trajectory and corresponding inputs may be used.

The interval of the trajectory depends on the robot's specifications (e.g., memory, motor resolution, etc.). However, the trajectory may be extended with interpolation methods up to submillimeter intervals. The trajectory is defined with waypoints. Additional waypoints may be created with a smaller interval by interpolation.

The trajectory is then stored for automatic or semi-automatic use. The trajectory may be indexed as one trajectory in a set of trajectories for later use or use with different patients. The trajectory is added to the library. Alternatively, the trajectory is used for the current patient.

The trajectory as created and/or used indicates the motor positions and/or imaging catheter positions. Using kinematics or other information, the motor positions of the robotic catheter system are related to the real-space or patient centric positions of the catheter. The trajectory is defined in either space or a different domain.

Figure 2 illustrates several paths 200 taken by or navigable by a catheter. The illustration is in two dimensions for simplicity, but the paths 200 are three-dimensional. Three different possible or used trajectories 202, 204, and 206 along different branches of the path 200 are shown. The trajectories 202, 204, and 206 are designated with start (triangle) and end (square) points. Any number of points (circles) may be acquired between the start and end points. The interval between points is defined in distance. Path 202 has a smaller interval, such as 1 mm. Path 204 has an interval of 5 mm. Path 206 has a 10 mm interval. Other intervals may be used, or a continuous trajectory may be used.

The trajectory includes the view vector, represented by the arrow at each point. The rotation and/or bending at each location may be used instead or with the view vector to form the trajectory.

In other implementations, the acquired trajectory was created without recording the survey or position of the catheter, at least for part of the trajectory. The processor receives a user designation of the trajectory on a user interface. The user inputs the way points, including the start or initial point and the end or last point. Using three-dimensional rendering, the user inputs on the user interface (e.g., display) waypoints in three-dimensional space as simulated or modeled.

In one approach, a representation of the patient anatomy is displayed. The representation includes a boundary. The boundary may be an anatomical boundary, such as the heart wall, a limitation on region of operation of the robotic catheter system, or a combination of both. The user inputs waypoints within the boundary. The sequence of the waypoints is indicated, such as by the order of input. By entry on the graphics user interface, the user creates the trajectory. The trajectory may be created as part of pre-planning and/or during a procedure. Alternatively, the trajectory is created without reference to a particular patient. The trajectory is created on the user interface as a possible safe path from the start to the end with waypoints being input.

In one implementation, the anatomy is actively mapped. The imaging catheter images the anatomy, such as a heart chamber. The trajectory is then created or defined with respect to this mapping. The map is in 6 degrees of freedom (DOF) (3-dof position, 3-dof orientation) for the user. Figure 3 shows an example where kinematics from the robotic catheter system with images acquired using the imaging catheter under the control of the robotic catheter system define the boundary 302 or reachable region. The user picks the 'start' and `end' positions on the visualization map (i.e., user interface) to define the trajectory 300. Then, waypoints are interpolated in 6-DOF based on the input within the bounded kinematics, which was constructed by the previous trace of the user's inputs. The basic concept is to construct a 3-D convex-hull on the 3D boundary 302 of the robot configuration using kinematics. The user can add additional way points. New trajectories are created between 'start' and 'end.' This can be labelled as a new trajectory for a shuttling motion.

The trajectory may be aligned with the patient using landmarks, position sensing (e.g., electromagnetic position sensing or x-ray imaging), or imaging from the catheter. Alternatively, the trajectory is acquired monitoring use of the catheter and/or operation of the robotic catheter system to survey or monitor the patient, so has an alignment based on that use.

In act 120 of Figure 1, the processor moves the catheter. The processor controls the robotic catheter system to move the catheter. For example, the processor causes the robotic catheter system to translate, rotate, and/or bend the catheter while part of the catheter is in the patient.

The movement is along the trajectory. The trajectory defines the translation, rotation, and/or bending. The processor causes the robotic catheter system to translate, rotate, and/or bend the catheter to move the catheter along the trajectory.

The movement is automatic or semi-automatic. The user can either set the robotic catheter system to automatically follow (e.g., continuously loop) along the chosen trajectory or have more in-depth control by semi-automatic moving step-by-step (e.g., one button press or activation at a time causes the robotic catheter system to move the catheter to the next waypoint or interval in the trajectory). After each activation, the robotic catheter system pauses the movement at the waypoint or interval along the trajectory.

In one implementation, the processor causes the robotic catheter system to repeat movement of the imaging catheter over the trajectory multiple times. Back and forth progression from the end to start and vice versa occurs for regular or continuous monitoring using the imaging catheter. The navigation of the imaging catheter along the trajectory between the start point and end point is performed without the operator performing the navigation. The user may start or activate the loop, but the robotic catheter system navigates the catheter along the trajectory with or without pauses at waypoints without further input or control by the user.

Physicians typically survey anatomy with ICE to view how devices interact or have deployed over a region. This surveying may be performed multiple times with continuous back-and-forth of the physician between manipulating the ICE to survey and adjusting any viewed device based on ICE feedback. By reproducing the motion of the initial survey using the robotic catheter system following the trajectory, hands-free repeat image surveying of anatomy and device deployment is provided. The user is free to focus more on the images and control of other devices. This anatomical survey is reproduced by traversing within this trajectory of motor configurations.

If capable, the robotic catheter system may move the catheter to the start or end point of the trajectory from any location within the patient. For example, an open loop control is used to move to a defined position. The trajectory may then be used for control of the catheter. Following initialization, the catheter is moved automatically to the trajectory, which then allows for the robotic catheter system to shuttle the catheter along the trajectory. Alternatively, the user causes the robotic catheter system to position the catheter along the trajectory (e.g., at one of the end points), and then movement is performed along the trajectory.

Manual control of the ICE catheter is tedious and can be time-consuming when considering that the interventionalist must stop handling therapeutic or device catheters to control the ICE imaging. The user can manually construct a library of interesting trajectories based on the trace or path of prior steps. Then, the user can automatically reproduce views or surveying along these trajectories with less interaction by processor-based control along the trajectory.

The user may set or change the interval used in the trajectory. Where the trajectory is continuous, the robotic catheter system continuously moves the catheter. Where waypoints are included, the robotic catheter system either continuously moves through the waypoints or pauses at each or designated waypoints. The interval may be adjusted by changing the step size. An interpolation may be implemented to generate fine steps of the specified trajectory (e.g., from A to B by n-step size given m-step size trajectory where n<m). The user can then initialize the robot to continuously loop through this trajectory until a stop command is given.

In another implementation, the movement is performed in stages. The robotic catheter system moves the catheter to waypoints along the trajectory, step-by-step. There is a pause at each waypoint until further movement is activated, a trigger occurs (such as based on heart motion), and/or a set period passes. The distance between waypoints is part of the trajectory and/or may be set by the user. For example, the user inputs 1, 2, 5, 10, or other number of millimeters for the distance between waypoints along the trajectory. Sub-millimeter motions may be included so that the user can see subtle changes in the field of view during ICE survey. The user fine-tunes the steps in a trajectory so that the user may easily locate a desired or missing view. Rough or large jumps in views may be avoided or limited by setting the step size or interval to be smaller. Alternatively, the waypoints have different spacing along the trajectory, such as the user assigning waypoints based on anatomy or location of other devices.

By moving between waypoints in response to activation by the user (e.g., user pressing an arrow or other button), a desired view may be found. For example, more precise ICE views are found. The robotic controller or processor can find the desired view by incrementing along the waypoints or trajectory rather than the user attempting to manually control the catheter to steer to the desired view. In one approach, the operator views the sequence of images captured along the trajectory and selects the desired view. The processor then controls the robotic catheter system to move along the trajectory to the position (translation, rotation, and bending) used to capture that view for generating further images in the desired view. This shuttling of the view allows the user to iterate in increments to achieve the optimal image-view needed for therapeutic delivery or other catheter operation.

This shuttling provides for reversing (end-to-start direction) or progressing (start-to-end direction) along the trajectory. In response to operator input, the processor causes the robotic catheter system to move the catheter along the trajectory. The operator may reverse to a point along the trajectory having a view indicated by an operator. The user can progress forward or backward within that trajectory - incrementing or decrementing the current state of the robot along the specified trajectory. For example, before performing a therapeutic task, physicians survey the anatomy by manually controlling the robotic catheter system to determine their preferred view for parking the ICE catheter. During this survey, the user may pass by an ideal view and be unable to return to it by hand - ultimately compromising for a less optimal view. Instead, the path for the survey is identified or created as a trajectory. The user may then increment along the trajectory, such as in reverse, for semi-automatic operation to find again the desired view and/or may select the desired view from the images so that the robotic catheter system automatically returns the imaging catheter to the position for the view. In either case, the processor causes a rewind of the survey to the preferred view for an instant replay.

In another approach, the processor causes the robotic catheter system to move the catheter in synchronization with heart motion. Using ECG or image processing, the heart cycle or trace is input to the processor or a trigger device. The robotic catheter system moves the catheter during a particular point or part of the heart cycle and/or moves the catheter for imaging or treatment at a particular point or part of the heart cycle. For example, certain techniques (e.g., mapping) are used to generate a 3D map of the heart chambers for procedural guidance. These mapping techniques can have error depending on sampling rate compared to cardiac motion. The robotic catheter system steps through intervals or waypoints in the mapping while accounting for heart motion. The imaging is gated to the cardiac cycle while imaging along the trajectory, and/or deformation correction is applied to the imaging or mapping where the amount and/or shape of deformation is based on the heart cycle and position along the trajectory.

For moving, the processor controls the motors of the robotic catheter system. The robotic catheter system, by actuation of the motors, manipulates the catheter. The manipulation may include steering (e.g., bending in one or more planes), global rotation (e.g., rotation of the handle and corresponding catheter), global translation (e.g., pulling or pushing the catheter further out or further into the patient), imaging, ablation, puncture, inflation, stitching, clamping, cutting, pharmaceutical deposit, stent expansion, balloon inflation, stent release, and/or another operation. The robot, under control of the processor, operates the catheters. Similarly, the user interface (e.g., display and input devices) of the robotic catheter system provides manual control of the robotic catheter system. The processor may receive the inputs and/or generate the outputs for the user interface and provide controls to the motors. The processor may generate controls for the motors based on programming for automated or semi-automated operation.

In act 130, a display displays one or more images from an imaging catheter. For ICE, the images are ultrasound. For an endoscope or catheter with an optical camera, the images are optical. The images are of the anatomy and/or other devices while the sensor (e.g., transducer array) is positioned along the trajectory. A sequence of images from one location or from different positions along the trajectory may be generated.

For non-imaging catheters, the catheter is used to apply treatment (e.g., ablation, stenting, suturing, cutting, pharmaceutical application, or another) or assistance (e.g., clamping, moving tissue, or another).

Figure 4 is a block diagram of one embodiment of a catheter control system. A control system using a robotic catheter system 400 is provided for a steerable catheter 450. The control system uses a trajectory for automatic or semi-automatic movement of the catheter 450 by the robotic catheter system 400, freeing the user to concentrate on imaging information and/or other operations than navigating the catheter 450.

The control system includes the robotic catheter system 400 formed by the base 440 and adaptor 442. The processor 410, memory 420, and/or display 430 are part of a separate computer, workstation, or server and/or are part of the robotic catheter system 400. Additional, different, or fewer components may be provided. For example, the base 440 and adaptor 442 are combined to be one housing for motors and clamps to manipulate the catheter 450.

The robotic catheter system 400 is configured for robotically controlling the catheters 450. In an example used herein, the robotic catheter system 400 is configured to control an ICE catheter 450. Other types of steerable catheters may be used, such as controlling an ablation catheter or another imaging catheter. Other catheter-like devices (e.g., fiber optic endoscope or needle) may be controlled. These different catheters may have the same or different arrangements of control knobs, steering, degrees of freedom, and/or operation, so the robotic catheter systems 400 is configured to operate or control the arrangements of the catheter 450 being used. Different catheter systems may be configured to operate with different types of catheters and not other types.

Any type or design of robotic catheter system 400 may be used. In the example herein, the robotic catheter system 400 includes the base 440 and an adaptor 442. The base 440 includes the motors for steering and/or operating the catheter 450, and the adaptor 442 is configured to mate with, hold, and/or manipulate the catheter 450. For example, the adaptor 442 allows the robotic catheter system 400 to operate the steerable ICE catheter 450 designed for manual control by a physician. U.S. Patent No. 11,590, 319, the disclosure of which is incorporated herein by reference, discloses various embodiments of such a robotic catheter system. Other designs may be used, such as robotic catheter systems designed to robotically control a catheter designed specifically for or to mate with and be controlled by the robot (e.g., no adaptor 442).

The base 440 includes motors to manipulate the steerable catheter 450 in any number of degrees of freedom, such as 4 or more degrees of freedom (e.g., global translation, global rotation, and bending or steering the catheter 450 in two or more directions or planes). Gearing, motors, grippers (clamps), connectors, and/or other robotic components are included the base 440 for generating and transmitting force to operate the catheter 450. The robotic catheter system 400 applies push and/or pull forces to steering wires 456, 458 (e.g., three or four steering wires per catheter 450) to operate the catheter 450. The tips are steered for catheter operation. Electrical signals, translation, and/or rotation force may be generated in the base 440. Global translation and/or rotation forces may similarly be applied, such as through the handles of the catheter 450.

The adaptor 442 connects or plugs to the base 440 and manipulates the catheter 450. In one embodiment, the adaptor 442 is a clamshell or other arrangement of housing, gearing, connectors, clamps, and/or other robotic components to transfer force from the base 440 to the catheter 450 to steer and/or otherwise operate the catheter 450. In one approach, the adaptor 442 fits around the handle and/or body of the catheter 450 to allow for manipulating the actuators (e.g., knobs) of the catheter 450, for instance AP/LR knobs of an ICE catheter and/or the handle of the catheter 450 for global translation and/or rotation (e.g., push-pull knob of an ablation catheter, etc.). Other adaptors 442 that support other degrees of freedom (e.g., 2, 3, 4, or more) may be used, such as for control of needle or ablation catheters.

Where the steerable catheter 450 is an ICE catheter, the robotic catheter system 400 may be configured to control the ICE catheter, including imaging. The array 452 of elements is controlled through beamforming to generate ultrasound images. The field of view 454, type of imaging, and/or other imaging control may be provided. Where the steerable catheter 450 is an ablation or other treatment catheter, the robotic catheter system 400 may be configured to control the ablation or treatment catheter. For example, the contact sensing and/or electrode or another ablation applicator is controlled to ablate or treat. The ablation control may be performed by the robotic catheter system 400 and/or the processor 410.

The memory 420 is a non-transitory memory, such as a removable storage medium, a random-access memory, a read only memory, a memory of a field programmable gate array, a cache, and/or another memory. The memory 420 is configured by a processor, such as the processor 410, to store a trajectory, images, waypoints, control signals, information, control software, instructions executable by the processor 410, instructions executable by the robotic catheter system 400, interface software, and/or other information. In one implementation, the memory 420 stores the trajectory as a sequence of motor configurations at different locations along a path, including starting location A, ending location B, and intervening locations recorded during creation of the trajectory. A library of trajectories may be stored.

The display/user interface 430 is a display screen, such as a liquid crystal display or monitor, and a user input, such as a keyboard, knobs, sliders, touchpad, mouse, and/or trackpad. The display/user interface 430 may include icons, graphics, or other imaging, such as from an operating system or application, for user interaction (selection, activation, and/or output).

The processor 410 is a general processor, application specific integrated circuit, integrated circuit, digital signal processor, field programmable gate array, artificial intelligence processor, tensor processor, and/or another controller for interfacing with or controlling the robotic catheter system 400, the user interface/display 430, and/or catheter 450. The processor 410 is configured by design, hardware, firmware, and/or software to interface between the user and robotic catheter system 400 and/or to control the robotic catheter system 400. Multiple processors may be used for sequential and/or parallel processing as the processor 410. The instructions from the memory 420, when executed by the processor 410, cause the processor 410 to operate the robotic catheter system 400 and/or the steerable catheter 450 and to interface between various components and/or with the user or operator.

The processor 410 is in a same room as the robotic catheter system 400. For example, a computer or workstation is connected with wires or wirelessly for interacting with the robotic catheter system 400. In one implementation, the processor 410 is a processor of the robotic catheter system 400. Alternatively, the processor 410 is in a different room than the robotic catheter system 400, such as in a room of the same building, different building, different facility, or different region. Computer network communications are used between the processor 410 and the robotic catheter system 400. The control through the processor 410 for the robotic catheter system 400 allows the processor 410 to be fully remote from the procedure, patient, and/or robotic catheter system 400 as the processor 410 provides for the catheter 450 to be manipulated robotically.

The trajectory is stored in the memory 420. As the operator controls the robotic system 400 to move the catheter 450 and/or the user interface 430 to enter waypoints, the trajectory is created and stored in the memory 420. The trajectory is created by tracking or storing manual operation of the robotic system 400, by trajectory design in a model of anatomy (e.g., created with a user interface receiving inputs of waypoints within a boundary for the patient from a survey), and/or another process.

The processor 410 is configured to cause the robotic system 400 to navigate the steerable catheter 450 using the trajectory. The trajectory defines the path and/or points along which or to which the robotic system 400 navigates the catheter 450.

In one implementation, the processor 410 is configured to cause the robotic system 400 to move the catheter 450 in a rewind along the trajectory. For example, the robotic system 400 (catheter robot) moves an ICE catheter 450 along a previously established trajectory for the patient to a location previously used.

In one approach, the movement or caused navigation along the trajectory is performed in steps. A step size is based on waypoints in the trajectory and/or based on an operation established or input interval (e.g., 1, 5, or 10 mm). The robotic system 400, under the control of the processor 410, causes the catheter 450 to move the next step in response to a trigger. The trigger is from the heart cycle, breathing cycle, identification or detection in an image, tracking of another device, and/or user activation. For example, the user presses a back arrow to increment along the trajectory in the end-to-start direction in rewind by one step. Pressing the forward arrow increments along the trajectory in the start-to-end direction in a progression by one step. In the rewind, the catheter 450 is moved in reverse along the trajectory. The rewind may be by one step and/or to a desired view, such as rewinding to a previous view selected as being of interest from a sequence of images from along the trajectory. Where a sequence of images is generated as the robotic system 400 navigates the imaging catheter 450 along the trajectory, the processor 410 is configured to cause the robotic system 400 to navigate the imaging catheter 450 to a point along the trajectory associated with one of the images in the sequence.

In another implementation, the processor 410 is configured to cause movement of the catheter 450 along part or the entire trajectory with or without pauses. The movement and imaging from the trajectory are repeated. Multiple repetitions may be provided, such as looping back and forth along the trajectory. In another approach, the robotic system 400 moves the catheter 450 from the end of the trajectory to the start along a path different than the trajectory.

In yet another implementation, the processor 410 is configured to cause movement of the catheter 450 in synchronization with a heart, breathing, or other physiological cycle. For example, an ECG signal or other cyclic signal is received or created. The processor 410 receives or uses the cyclic signal to control the movement. The movement occurs at some parts of the cycle and not others. Alternatively, or additionally, the processor 410 controls operation (e.g., gating imaging) of the catheter by the robotic system 400 based on the cycle.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, including all equivalents, that are intended to define the spirit and scope of this invention.

## Claims

1. A method for robotic control of an imaging catheter (450), the method comprising:
acquiring (110) a trajectory (202, 204, 206) of catheter (450) movement within a patient;
moving (120) the imaging catheter (450) by a robotic catheter system (400), the moving (120) being along the trajectory (202, 204, 206); and
displaying (130) an image from the imaging catheter (450), the image of the patient while the imaging catheter (450) is on the trajectory (202, 204, 206).

2. The method of claim 1, wherein acquiring (110) comprises receiving an input of a start point and an input of an end point from an operator navigating the imaging catheter (450) along the trajectory (202, 204, 206) with the robotic catheter system (400), and wherein moving (120) comprises repeating the navigation of the imaging catheter (450) along the trajectory (202, 204, 206) between the start point and end point without the operator performing the navigation.

3. The method of claim 1, wherein acquiring (110) comprises storing the trajectory (202, 204, 206) during manual control by an operator of the robotic catheter system (400).

4. The method of claim 3, wherein storing comprises storing the trajectory (202, 204, 206) as motor positions or imaging catheter (450) positions.

5. The method of claim 1, wherein acquiring (110) comprises receiving a user designation of the trajectory (202, 204, 206).

6. The method of claim 5, wherein receiving the user designation comprises receiving user input of way points within a boundary on a graphics user interface.

7. The method of claim 1, wherein moving (120) comprises moving (120) the imaging catheter (450) to waypoints on the trajectory (202, 204, 206) in response to activation for each of the waypoints by the user, an interval of the waypoints input by an operator.

8. The method of claim 7, wherein the waypoints comprise one or more waypoints interpolated from others of the waypoints.

9. The method of claim 1, wherein moving (120) comprises repeating movement of the imaging catheter (450) over the trajectory (202, 204, 206) multiple times.

10. The method of claim 1, wherein moving (120) comprises reversing or progressing along the trajectory (202, 204, 206) in response to operator input.

11. The method of claim 10, wherein reversing comprises reversing to a point along the trajectory (202, 204, 206) having a view indicated by an operator.

12. The method of claim 1, wherein moving (120) comprises moving (120) by the robotic catheter system (400) in synchronization with heart motion.

13. The method of claim 1, further comprising receiving (100) user selection of the trajectory (202, 204, 206) from a library of trajectories.

14. The method of claim 1, wherein moving (120) comprises moving (120) the imaging catheter (450) at an interval between waypoints, the interval input by an operator.

15. A control system for a steerable catheter (450), the control system comprising:
a robotic system (400) for operation of a steerable catheter (450);
a memory (420) configured to store a trajectory (202, 204, 206) along which the robotic system (400) navigates the steerable catheter (450); and
a processor (410) configured to cause the robotic system (400) to navigate the steerable catheter (450) in a rewind along the trajectory (202, 204, 206).

16. The control system of claim 15, wherein the processor (410) is configured to cause the navigation along the trajectory (202, 204, 206) in steps, a step size configured by input from an operator and the navigation for each step being in response to user activation.

17. The control system of claim 15, wherein the steerable catheter (450) comprises an imaging catheter where a sequence of images is generated as the robotic system (400) navigates the imaging catheter along the trajectory (202, 204, 206), and wherein the processor (410) is configured to cause the robotic system (400) to navigate the imaging catheter to a point along the trajectory (202, 204, 206) associated with one of the images in the sequence.

18. The control system of claim 15, wherein the processor (410) is configured to store the trajectory (202, 204, 206) in the memory (420) as an operator controls the robotic system (400).

19. The control system of claim 15, wherein the processor (410) is configured to store the trajectory (202, 204, 206) in the memory (420), the trajectory (202, 204, 206) created with a user interface (430) receiving input of waypoints within a boundary.

20. A catheter control system comprising:
a memory (420) configured to store instructions; and
a processor (410) configured by the instructions to control a catheter (450) robot to move an intracardiac echocardiography catheter (450) along a previously established trajectory (202, 204, 206).

21. The catheter control system of claim 20, wherein the processor (410) is configured to control the catheter (450) robot to move the intracardiac echocardiography catheter (450) in reverse along the trajectory (202, 204, 206) as a rewind to a previous view.
